Europäisches Patentamt

European Patent Office

Office européen des brevets

(11) Veröffentlichungsnummer : **0 355 664 B1**

(12) **EUROPÄISCHE PATENTSCHRIFT**

(45) Veröffentlichungstag der Patentschrift :
**15.04.92 Patentblatt 92/16**

(21) Anmeldenummer : **89115026.0**

(22) Anmeldetag : **15.08.89**

(51) Int. Cl.⁵ : **B01J 35/02,** B01J 23/31,
B01J 27/192, B01J 35/04

(54) **Katalysator für die Oxidation und Ammonoxidation von alpha-, beta-ungesättigten Kohlenwasserstoffen.**

Die Akte enthält technische Angaben, die nach dem Eingang der Anmeldung eingereicht wurden und die nicht in dieser Patentschrift enthalten sind.

(30) Priorität : **16.08.88 DE 3827639**

(43) Veröffentlichungstag der Anmeldung :
**28.02.90 Patentblatt 90/09**

(45) Bekanntmachung des Hinweises auf die Patenterteilung :
**15.04.92 Patentblatt 92/16**

(84) Benannte Vertragsstaaten :
**DE ES FR GB IT**

(56) Entgegenhaltungen :
**EP-A- 0 102 641**
**EP-A- 0 141 997**
**EP-A- 0 220 933**
**US-A- 4 337 178**

(73) Patentinhaber : **BASF Aktiengesellschaft**
**Carl-Bosch-Strasse 38**
**W-6700 Ludwigshafen (DE)**

(72) Erfinder : **Martan, Hans, Dr.**
**Mozartstrasse 62**
**W-6710 Frankenthal (DE)**
Erfinder : **Mross, Wolf Dieter, Dr.**
**Anselm-Feuerbach-Strasse 21**
**W-6710 Frankenthal (DE)**
Erfinder : **Engert, Gerd-Juergen, Dr.**
**Muehlaustrasse 4**
**W-6700 Ludwigshafen (DE)**

## Beschreibung

Die Erfindung betrifft Katalysatorteilchen aus einer aktiven Masse, wie sie als Katalysatoren für die Oxidation und Ammonoxidation von $\alpha$-, $\beta$-olefinische ungesättigten Kohlenwasserstoffen zu den entsprechenden Aldehyden, Carbonsäuren oder Nitrilen eingesetzt werden.

Für die Herstellung von $\alpha$-, $\beta$-ungesättigten Aldehyden, Carbonsäuren und Nitrilen durch Oxidation oder Ammonoxidation von olefinisch ungesättigten Kohlenwasserstoffen in der Gasphase bei Temperaturen von etwa 250 bis 450°C, oft in Gegenwart von Wasserdampf, wurden schon eine Vielzahl von oxidischen Katalysatoren vorgeschlagen, die sich meist von überwiegenden Anteilen an Molybdän (und/oder Vanadin und/oder Wolfram) und untergeordneten Mengen an Bismut, sowie Eisen (und/oder Cer) und Nickel und/oder Kobalt und-/oder Zink sowie Spuren an Alkalimetallen, bevorzugt Kalium, ableiten und die zusätzlich Phosphor, Arsen, Antimon, Bor sowie ferner (oft als Bestandteile von Trägermaterialien), Silicium, Aluminium und/oder Titan in oxidischer Form enthalten. Derartige Katalysatoren werden häufig in Form von Zylindern sowie von Ringen oder Kugeln eingesetzt, wobei die katalytisch aktive Masse oft auf kugelförmige oder ringförmige Träger in dünner Schicht aufgebracht ist. Katalysatoren dieser Art sind z.B. in der DE-OS 33 00 044, der deutschen Patentschrift 22 49 922 und den europäischen Patentschriften 15 565, 15 569, 17 000 68 192 und 102 641 beschrieben. Die Katalysatoren dieser Art werden bei den Oxidations- bzw. Ammonoxidationsreaktionen im allgemeinen als Festbettkatalysatoren eingesetzt.

Bei der praktischen Durchführung dieser Oxidationsverfahren, beispielsweise zur Herstellung von Acrolein und Acrylsäure durch Gasphasenoxidation von Propylen lassen jedoch die erhaltenen Ausbeuten und Selektivitäten immer noch zu wünschen übrig. So weisen zwar Vollkatalysatoren, d.h. Katalysatoren deren Volumen vollständig aus der ggf. durch Trägermaterialien verdünnten katalytisch aktiven Masse hergestellt sind, eine hohe volumenspezifische Aktivität auf doch sind sie verhältnismäßig unselektiv, führen also zu einer Weiteroxidation zu den thermodynamisch begünstigten Endprodukten CO und $CO_2$. Günstiger in dieser Hinsicht sind Schalenkatalysatoren, d.h. Katalysatoren bei denen ein (meist kugelförmiger) Kern aus katalytisch inaktivem Trägermaterial eine dünne Schale aus dem katalytisch aktiven Material aufweist.

Diese weisen zwar eine verhältnismäßig gute Selektivität auf, zeigen jedoch nur geringe volumenspezifische Aktivität, da der größte Teil des Volumens durch katalytisch inaktives Trägermaterial ausgefüllt ist. Sie müssen daher bei verhältnismäßig hohen Temperaturen betrieben werden, so daß die Katalysatorstandzeiten beeinträchtigt und die Selektivität nicht optimal genutzt wird. Zudem weisen sie häufig, wie auch die meisten Vollkatalysatoren, einen beachtlichen Strömungswiderstand auf. Eine Erhöhung des Drucks bei den genannten Oxidations- und Ammonoxidationsverfahren begünstigt jedoch gleichfalls die zu Kohlenoxid und Kohlendioxid führenden Nebenreaktionen.

Aufgabe der vorliegenden Erfindung sind nun Katalysatoren mit gleichzeitig besonders hohen Selektivitäten und volumenspezifischen Aktivitäten für die Gasphasenoxidation und Ammonoxidation von monoolefinisch ungesättigten Kohlenwasserstoffen zu $\alpha,\beta$-monoolefinisch ungesättigten Aldehyden, $\alpha,\beta$-monoolefinisch ungesättigten Carbonsäuren und $\alpha,\beta$-monoolefinisch ungesättigten Nitrilen.

Dieses Ziel wurde mit Katalysator-Teilchen einer aktiven Masse der allgemeinen Formel

$$[Mo,V,W]_{12}Bi_a[Fe,Ce]_b[Ni,Co,Zn]_c[Alkali]_d[P,As,Sb,B]_e[Si,Al,Ti]_fO_x$$

in der

| | | |
|---|---|---|
| a | 0,1 | bis 5, |
| b | 0,1 | bis 10, |
| c | 1 | bis 15, |
| d | 0,01 | bis 2, |
| e | 0 | bis 2, |
| f | 0 | bis 30 und |
| x | die zur Absättigung der Valenzen der anderen Komponenten erforderliche Zahl der Sauerstoffatome bedeuten, | |

dadurch erreicht, daß sie die Form eines 3 bis 5-speichigen Rades oder einer Rosette aufweisen, die Wandstärken von 0,5 bis 4 mm und einen Durchmesser von 3 bis 20 mm haben.

Die katalytisch aktiven Massen der angegebenen allgemeinen Formel können in an sich üblicher und bekannter Weise durch Vermischen wäßriger Lösungen von vorzugsweise leicht zersetzlichen Salzen der Komponenten, Eindampfen der Gemische und/oder Sprühtrocknen, ggf. Verdichten und ggf. mehrstufigen Kal-

2

zinieren meist bei Temperaturen von 250 bis 700°C, vorzugsweise in zwei Stufen bei Temperaturen von 250 bis 400°C und von 480 bis 660°C sowie Vermahlen auf Teilchengrößen, die im allgemeinen 0,1 bis 300, vorzugsweise 0,2 bis 150, insbesondere 0,5 bis 50 µm betragen, hergestellt werden. Dabei ist es von besonderem Vorteil, daß man für die Herstellung der Katalysatoren Eisenmolybdat-Gel oder Bismutwolframat in getrockneter, pulverförmiger Form einsetzt. Ein derartiges Gel kann z.B. nach den Angaben der GB-PS'en 1 282 949 und 1 282 950 hergestellt sein. Der Gehalt der katalytisch aktiven Masse an Bismut und Wolfram kann dem Gemisch der wasserlöslichen Salze der anderen Komponenten in Form von Bismut-Wolframat zugegeben werden. Bei der Herstellung der erfindungsgemäßen Katalysator-Teilchen kann die katalytisch aktive Masse der angegebenen allgemeinen Formel auch durch Trägermaterialien wie Kieselgur, kolloidales Siliciumdioxid (Kieselsol) oder feinteiliges Titandioxid in an sich üblicher Weise verdünnt sein, wobei der Anteil an derartigen Trägermaterialien bis zu 50 Gew.-Teile je 100 Gew.-Teile katalytisch aktiver Masse betragen kann. Meist wird jedoch vorgezogen, eine wenig verdünnte katalytisch aktive Masse für die Herstellung der erfindungsgemäßen Katalysator-Teilchen einzusetzen.

Zur Herstellung der neuen Katalysator-Teilchen wird im allgemeinen aus der pulverförmigen katalytisch aktiven Masse, die kalziniert oder nicht kalziniert sein kann, ggf. feinteiligem Trägermaterial sowie Verformungshilfsstoffen, wie Stearinsäure, Ruß und Melamin sowie Wasser (meist 100 bis 500 Teile auf 100 Teile Feststoffe) eine breiartige Masse, hergestellt aus der die Formen beispielsweise durch Extrudieren und Abschneiden von Scheiben des Extrudats geformt werden. Die neuen Katalysatorteilchen können auch aus der katalytisch aktiven Masse und ggf. Zusatzstoffen der genannten Art durch Pressen hergestellt werden. Nach der Formgebung kann dann in an sich üblicher Weise kalziniert werden. Die neuen Katalysator-Teilchen haben insbesondere bei der Gasphasenoxidation von Propylen zu Acrolein und von Acrolein zu Acrylsäure besonders hohe volumenspezifische Aktivität und Selektivität und große mechanische Stabilität. Bei ihrem praktischen Einsatz zur Gasphasenoxidation oder zur Gasphasenammonoxidation insbesondere von Propylen, aber auch zur Gasphasenoxidation von Isobutylen zu Methacrolein und von Methacrolein zu Methacrylsäure tritt ein besonders geringer Druckabfall in den Reaktoren ein und der Temperaturverlauf wird durch verbesserte Verwirbelung der Reaktionsphase optimiert. Beim Einsatz der neuen Katalysatorteilchen können daher Reaktionsrohre mit verhältnismäßig großem freien Durchmesser sowie auch Katalysatorteilchen mit verhältnismäßig großen äußeren Abmessungen eingesetzt werden ohne daß dabei wegen mangelnder Wärmeabfuhr zu hohe Hot-Spots entstehen. Außerdem zeigen die neuen Katalysatorteilchen eine verbesserte Katalysatorstandzeit.

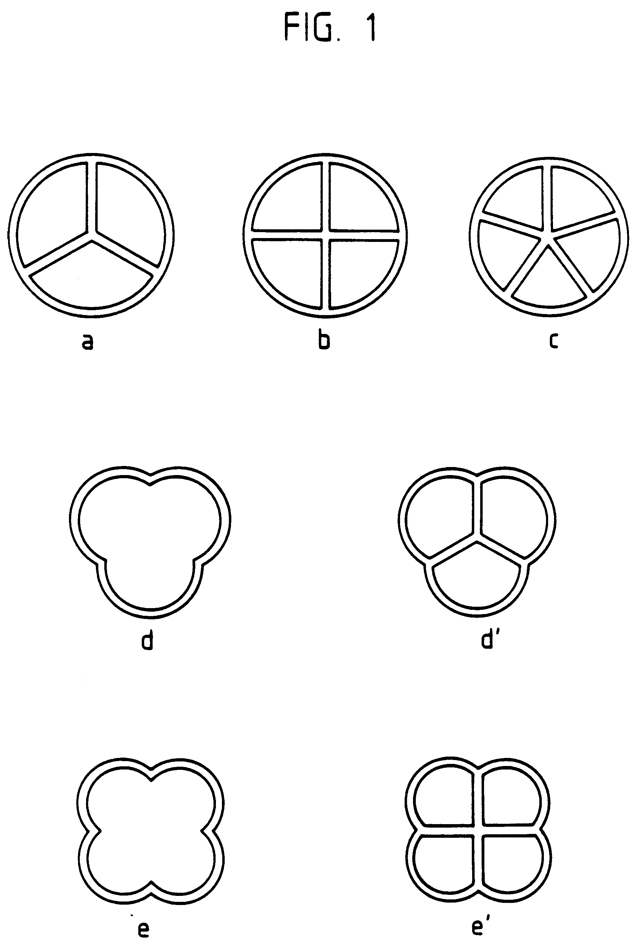

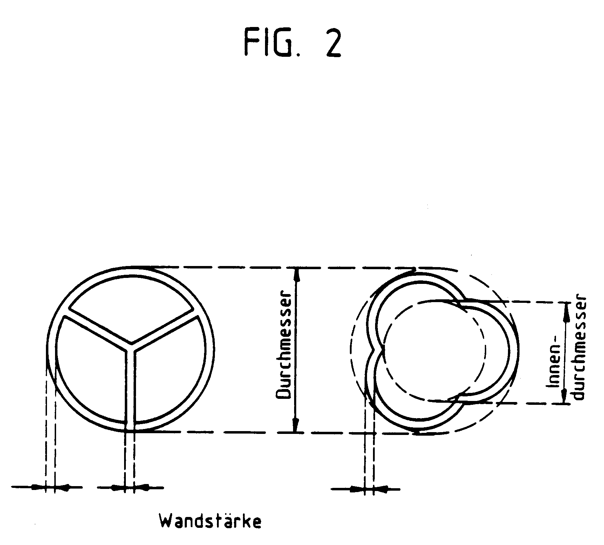

Die neuen Katalysatorteilchen werden durch die Abbildung 1 beispielhaft dargestellt, wobei (a) bis (c) 3 bis 5-speichige Räder, die Abbildungen (e) und (d) Rosetten ohne Speichen und die Abbildungen d' und e' Rosetten mit Speichen darstellen. In Abbildung 2 werden "Durchmesser" und "Wandstärken" der Räder und Rosetten sowie der "innere Durchmesser" der Rosetten schematisch dargestellt. Bei den Rosetten soll das Verhältnis von Durchmesser : innerem Durchmesser im allgemeinen im Bereich von 4 : 1 bis 1,2 : 1, vorzugsweise im Bereich von 2 : 1 bis 1,5 : 1 liegen.

Die in den folgenden Beispielen angegebenen Teile sind Gewichtsteile. Die darin angegebenen Volumenteile verhalten sich zu den Gewichtsteilen wie das Liter zum Kilogramm.

Beispiel 1:

Eine katalytisch aktive Masse der allgemeinen Formel

$$Bi_{1,39} W_{2,77} Mo_{9,23} Co_{3,85} Fe_{1,92} K_{0,08} Si_{1,23} O_x$$

wird nach den Angaben von Beispiel 1 der DOS 33 38 380 hergestellt und nach dem Trocknen auf eine Formgröße von 50 µm gemahlen. Man setzt dann auf 100 Teile dieser katalytisch aktiven Masse 20 Teile Wasser zu, verknetet zu einem Brei und extrudiert 3-speichige Räder (entsprechend Abb. 1a) mit einem Durchmesser von 5 mm und einer Wandstärke von 1 mm. Die Katalysatorteilchen werden dann getrocknet und bei 450°C 6 Stunden getempert.

40 Teile des erhaltenen Katalysators werden in einen mit einem Salzbad beheizten Reaktor eines Rohrinnendurchmessers von 12 mm eingefüllt. Der Reaktor wird bei einer Salzbadtemperatur von 360°C mit einer Mischung aus 4 Vol.-Teilen je Stunde Propen, 36 Vol.-Teile je Stunde Luft und 40 Vol.-Teilen je Stunde Stickstoff beschickt. Dabei stellt sich ein Propenumsatz von 95 Mol.-% und eine Ausbeute an Acrolein und Acrylsäure von zusammen 89 Mol.-% ein.

Vergleichsbeispiel 1:

Man arbeitet wie in Beispiel 1 angegeben, formt jedoch aus der angeteigten Katalysatormasse Ringe mit 5 mm Außendurchmesser und 1 mm Wandstärke. Unter sonst gleichen Bedingungen stellt sich in dem Reaktor ein Propenumsatz von 95 Mol.-% ein und die Ausbeute an Acrolein und Acrylsäure beträgt zusammen nur 86,6 Mol.-%.

Beispiel 2:

In einen Reaktor mit einem freien Rohrdurchmesser von 25 mm gibt man 1000 Teile des nach den Angaben in Beispiel 1 hergestellten Katalysators. Man heizt dann das Salzbad auf 320°C auf und leitet über den Katalysator je Stunde 120 Vol.-Teile Propen, 1080 Vol.-Teile Luft und 1200 Vol.-Teile eines inerten Gases ($N_2$). Der Druck am Reaktoreingang beträgt 1,6 bar und man erzielt einen Propenumsatz von 98 Mol.-%. Die Ausbeute an Acrolein und Acrylsäure beträgt zusammen 91,5 Mol.-%.

Beispiel 3:

Wie in Beispiel 1 angegeben stellt man 3-speichige Räder eines Außendurchmessers von 5 mm und einer Wandstärke von 1 mm aus einer katalytisch aktiven Katalysatormasse der allgemeinen Formel

$$Bi_{1,39}W_{2,77}Mo_{9,23}Co_{3,85}Fe_{1,92}K_{0,15}Si_{1,23}O_x$$

her und tempert diese wie in Beispiel 1 angegeben.

50 Teile dieser Katalysatorteilchen werden in einen mit einem Salzbad beheizten Reaktor eines Rohrinnendurchmessers von 12 mm eingefüllt. Bei einer Salzbadtemperatur von 360°C wird über die Katalysatorfüllung ein Gemisch aus 4 Vol.-Teilen je Stunde Propen, 36 Vol.-Teile je Stunde Luft und 40 Vol.-Teile je Stunde Stickstoff geleitet. Dabei stellt sich ein Propenumsatz von 95 Mol.-% ein und die Ausbeute an Acrolein und Acrylsäure beträgt zusammen 89 Mol.-%.

Beispiel 4:

Wie in Beispiel 1 angegeben stellt man 3-speichige Räder eines Außendurchmessers von 5 mm und einer Wandstärke von 1 mm aus einer katalytisch aktiven Masse der allgemeinen Formel

$$Bi_{1,39}W_{2,77}Mo_{9,23}Co_{3,85}Fe_{1,92}K_{0,31}So_{1,23}O_x$$

her und tempert diese wie in Beispiel 1 angegeben.

50 Teile dieser Katalysatorteilchen werden in einen mit einem Salzbad beheizten Reaktor eines Rohrinnendurchmessers von 12 mm eingefüllt. Über die Katalysatorfüllung leitet man bei einer Salzbadtemperatur von 360°C je Stunde ein Gemisch aus 3,8 Vol.-Teilen Propen, 34,2 Vol.-Teilen Luft und 38 Vol.-Teilen Stickstoff. Dabei stellt sich ein Propenumsatz von 95 Mol.-% ein und die Ausbeute an Acrolein und Acrylsäure beträgt zusammen 89 Mol.-%.

**Patentansprüche**

1. Katalysatorteilchen einer aktiven Masse der allgemeinen Formel

$$[Mo,V,W]_{12}Bi_a[Fe,Ce]_b[Ni,Co,Zn]_c[Alkali]_d[P,As,Sb,B]_e[Si,Al,Ti]_fO_x$$

in der
a 0,1 bis 5
b 0,1 bis 10
c 1 bis 15
d 0,01 bis 2
e 0 bis 2
f 0 bis 30 und
x die zur Absättigung der Valenzen der anderen Komponenten erforderliche Zahl der Sauerstoffatome bedeuten,
dadurch gekennzeichnet, daß sie die Form eines 3 bis 5-speichigen Rades, einer Rosette ohne Speichen oder einer Rosette mit Speichen aufweisen, die Wandstärken von 0,5 bis 4 mm und einen Durchmesser von 3 bis 20 mm haben, und bei den Rosetten mit oder ohne Speichen das Verhältnis von Durchmesser: innerem Durchmesser im Bereich von 4:1 bis 1,2:1 liegt.

4

**Claims**

1. A catalyst particle of an active material of the formula

$$[Mo,V,W]_{12}Bi_a[Fe,Ce]_b[Ni,Co,Zn]_c[Alkali]_d[P,As,Sb,B]_e[Si,Al,Ti]_fO_x$$

where
a is from 0.1 to 5,
b is from 0.1 to 10,
c is from 1 to 15,
d is from 0.01 to 2,
e is from 0 to 2,
f is from 0 to 30 and
x is the number of oxygen atoms required to saturate the valences of the other components,
wherein said particle has the shape of a 3-spoked to 5-spoked wheel or of a rosette without spokes or a rosette with spokes, which have wall thicknesses of from 0.5 to 4 mm and a diameter of from 3 to 20 mm and in the case of rosettes with or without spokes the ratio of diameter to internal diameter is from 4:1 to 1.2:1.

**Revendications**

1. Particules de catalyseur, faites d'une masse active de formule générale

$$[Mo,V,W]_{12}Bi_a[Fe, Ce]_b[Ni,Co,Zn]_c[Alcali]_d[P,As,Sb,B]_e[Si,Al,Ti]_fO_x$$

dans laquelle
a est un nombre de 0,1 à 5,
b est un nombre de 0,1 à 10,
c est un nombre de 1 à 15,
d est un nombre de 0,01 à 2,
e est un nombre de 0 à 2,
f est un nombre de 0 à 30 et
x est le nombre des atomes d'oxygène nécessaire pour la saturation des valences des autres composants,
caractérisé en ce qu'elles présentent la forme d'une roue comportant 3 à 5 rayons, d'une rosette sans rayons ou d'une rosette à rayons qui a une épaisseur de paroi de 0,5 à 4 mm et un diamètre de 3 à 20 mm, le rapport du diamètre au diamètre intérieur dans le cas des rosettes avec ou sans rayons se situant dans la gamme de 4:1 à 1,2:1.

# FIG. 1

a

b

c

d

d'

e

e'

# FIG. 2